# EUROPÄISCHE PATENTSCHRIFT

(11) **EP 1 309 607 B1**
(45) Veröffentlichungstag und Bekanntmachung des Hinweises auf die Patenterteilung: **27.10.2004**
(21) Anmeldenummer: 01978273.9
(22) Anmeldetag: 09.08.2001
(51) Int. Cl.: C07K 7/23, A61K 38/09

(54) **VERFAHREN ZUR HERSTELLUNG VON LHRH-ANTAGONISTEN**
METHOD FOR PRODUCING LHRH ANTAGONISTS
PROCEDE POUR PRODUIRE DES ANTAGONISTES DE LHRH

(30) Priorität: 17.08.2000 DE 10040700
(43) Veröffentlichungstag der Anmeldung: 14.05.2003
(73) Patentinhaber: Zentaris GmbH, 60314 Frankfurt/Main (DE)
(72) Erfinder: DAMM, Michael, 63322 Rödermark (DE); SALONEK, Waldemar, 69124 Heidelberg (DE); ENGEL, Jürgen, 63755 Alzenau (DE); BAUER, Horst, 91217 Hersbruck (DE); STACH, Gabriele, 60433 Frankfurt (DE)
(86) Internationale Anmeldenummer: PCT/EP2001/009219
(87) Internationale Veröffentlichungsnummer: WO 2002/014347

(56) Entgegenhaltungen:
- EP-A- 0 626 170
- WO-A-99/36099
- DE-A- 4 342 092
- LOTTSPEICH F & ZORBAS H: "Bioanalytik" 1998 , SPEKTRUM AKADEMISCHER VERLAG GMBH , HEIDELBERG, BERLIN XP002196554 Seite 201 -Seite 204
- NEUMÜLLER O-A: "Römpps Chemie-Lexikon" 1983 , FRANCKH'SCHE VERLAGSBUCHHANDLUNG , STUTTGART XP002196555 Seite 1923, linke Spalte

## Beschreibung

Gegenstand der vorliegenden Erfindung ist ein Verfahren zur Herstellung einer pharmazeutischen Zubereitung zur parenteralen Applikation bei Säugetieren dadurch gekennzeichnet, daß ein Säureadditionssalz eines basischen peptidischen LHRH-Antagonisten (Ausgangspeptidsalz) (1) in Gegenwart eines geeigneten Verdünnungsmittels mit einem Mischbettionenaustauscher oder einem Gemisch von einem sauren und basischen Ionenaustauscher unter Bildung des freien basischen Peptids umgesetzt, anschließend der Ionenaustauscher abgetrennt und dann das freie basische Peptid mit einer anorganischen oder organischen Säure unter Bildung des gewünschten Säureadditionssalzes des Peptids (Endpeptidsalz) (2) umgesetzt wird, dann geeignete pharmazeutische Hilfs-, Träger und/oder Gerüststoffe zugesetzt werden und anschließend das Verdünnungsmittel entfernt wird.

In der internationalen Patentanmeldung Nr. WO 95/15767 ist die Herstellung eines schwerlöslichen Peptides durch Umsetzung von einer wäßrigen Lösung des Säuresalzes mit einer essigsauren Lösung des basischen Peptids unter Ausfällung des schwerlöslichen Säureadditionssalzes des Peptids beschrieben. Beispielsweise wird die Herstellung des LHRH Antagonisten Cetrorelix Embonat beschrieben.

Der Ansdruck "basische peptidische LHRH-Antagonisten" bedeutet hier Antide, A-75998, Ganirelix, NalGlu-Antagonist, Cetrorelix, Teverelix (Antarelix ®) sowie die Antagonisten gemäß den Verbindungen: die aus der Patentanmeldung US 5 942 493 bekannten Verbindungen der allgemeinen Formel (I) 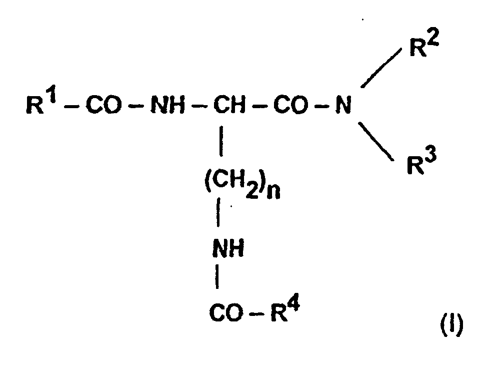 worin n die Zahl 3 oder 4, R¹ eine Alkylgruppe, eine Alkyloxygruppe, eine Arylgruppe, eine Heteroarylgruppe, eine Aralkylgruppe, eine Heteroaratkytgruppe, eine Aralkyloxygruppe oder eine Heteroaralfcytoxygruppe, jeweils unsubstitiert oder substituiert, R² und R³ unabhängig voneinander jeweils ein Wasserstoffatom, eine Alkylgruppe, eine Aralkylgruppe oder eine Heteroaralkylgruppe, jeweils unsubstituiert oder substituiert, bedeuten, wobei die Substitution wiederum aus einer Arylgruppe oder Heteroarylgruppe bestehen kann, oder -NR²R³ eine Aminosäuregruppe bedeutet, sowie R⁴ eine Gruppe mit der Formel (II) 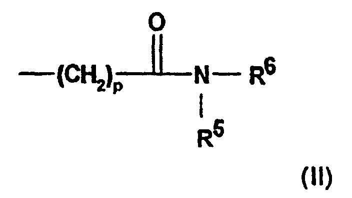 darstellt, in der p eine ganze Zahl von 1 bis 4, R⁵ Wasserstoff oder eine Alkylgruppe und R⁶ eine unsubstituierte oder substituierte Arylgruppe oder Heteroarylgruppe bedeutet, oder R⁴ einen Ring der allgemeinen Formel (III) 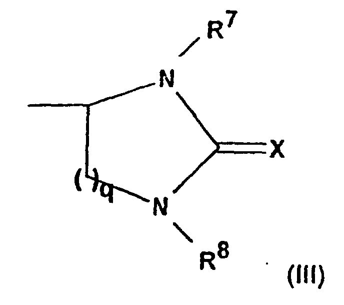 darstellt, in der q die Zahl 1 oder 2, R⁷ ein Wasserstoffatom oder eine Alkylgruppe, R⁸ ein Wasserstoffatom oder eine Alkylgruppe und X ein Sauerstoff- oder Schwefelatom bedeutet, wobei die aromatischen oder heteroaromatischen Reste teilweise oder vollständig hydriert vorliegen können sowie chirale Kohlenstoffatome R-oder S-konfiguriert sein können, und deren Salze mit pharmazeutisch akzeptablen Säuren,

Verbindung der allgemeinen Formel V

Ac-D-Nal(2)¹-D-(pCl)Phe²-D-Pal(3)³-Ser⁴-Tyr⁵-D-Xxx⁶-Leu⁷-Arg⁸-Pro⁹-D-Ala¹⁰-NH₂ (V)

worin D-Xxx eine Aminosäuregruppe der allgemeinen Formel (VI) 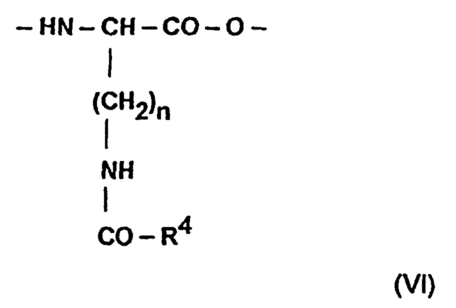 darstellt,
worin n die Zahl 3 oder 4, R⁴ eine Gruppe mit der Formel (II) 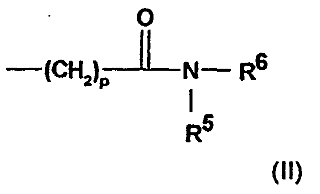 darstellt, in der p eine ganze Zahl von 1 bis 4, R⁵ Wasserstoff oder eine Alkylgruppe und R⁶ eine unsubstituierte oder substituierte Arylgruppe oder Heteroarylgruppe bedeutet,
oder R⁴ einen Ring der allgemeinen Formel (III) 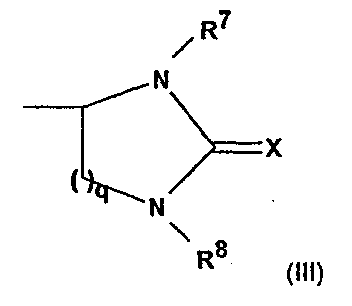 darstellt, in der q die Zahl 1 oder 2, R⁷ ein Wasserstoffatom oder eine Alkylgruppe, R⁸ ein Wasserstoffatom oder eine Alkylgruppe und X ein Sauerstoff- oder Schwefelatom bedeutet, und deren Salze mit pharmazeutisch akzeptablen Säuren, und die in der Patentanmeldung DE 19911771 offenbarten Verbindungen der folgenden allgemeinen Formel (I)

A-Xxx¹-Xxx²-Xxx³-Xxx⁴-Xxx⁵-Xxx⁶-Xxx⁷-Xxx⁸-Xxx⁹-Xxx¹⁰-NH₂ (I)

worin
A eine Acetyl- oder eine 3-(4-Fluorphenyl)-propionyl-Gruppe,
Xxx¹ D-Nal(1) oder D-Nal(2),
Xxx²-Xxx³ D-Cpa-D-Pal(3) oder eine Einfachbindung,
Xxx⁴ Ser,
Xxx⁵ N-Me-Tyr,
Xxx⁶ D-Cit, D-Hci oder eine D-Aminosäuregruppe der allgemeinen Formel (II) 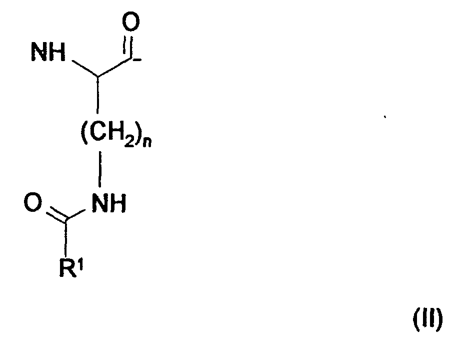 in der n die Zahl 3 oder 4 bedeutet, darstellt, wobei R¹ eine Gruppe mit der allgemeinen Formel III,

-(CH₂)ₚ-CO-NR²R³ (III)

worin p eine ganze Zahl von 1 bis 4, R² Wasserstoff oder eine Alkylgruppe und R³ eine unsubstituierte oder substituierte Arylgruppe oder Heteroarylgruppe bedeutet, oder R¹ eine 3-Amino-1,2,4-triazol-5-carbonyl-Gruppe oder R¹ einen Ring der allgemeinen Formel (IV) 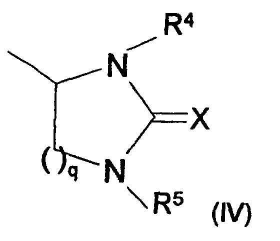 in dem q die Zahl 1 oder 2, R⁴ ein Wasserstoffatom oder eine Alkylgruppe, R⁵ ein Wasserstoffatom oder eine Alkylgruppe und X ein Sauerstoff- oder Schwefelatom bedeutet, darstellt,
Xxx⁷ Leu oder Nle,
Xxx⁸ Arg oder Lys(iPr),
Xxx⁹ Pro und
Xxx¹⁰ Ala oder Sar bedeutet,
und deren Salze mit pharmazeutisch akzeptablen Säuren,

Weitere Peptide sind Abarelix, Azaline B, Detirelix, Ramorelix (Stoeckemann and Sandow, J. Cancer Res. Clin. Oncol.1993, 119, 457) und RS-68439. Die Strukturen der genannten Peptide finden sich in: Behre et al., *GnRH antagonists: an overview*, Proceedings of the 2^{nd} World Conference on Ovulation Induction, The Parthenon Publishing Group Ltd.; Kutscher et al., Angew. Chem. 1997,109,2240.

Bei den als Edukte eingesetzten Säureadditionssalze der erfindungsgemäßen Peptide handelt es sich vorzugsweise um leichtlösliche Salze wie die Acetate, Hydrochloride oder Sulfate.

Gemäß dem erfindungsgemäßen Verfahren wird das Ausgangspeptidsalz in einem Verdünnungsmittel ganz oder teilweise gelöst oder darin suspendiert. Anschließend wird ein Verdünnungsmittel zugegeben. Lösungs- und Verdünnungsmittel können gleich oder verschieden sein. Als Lösungs- bzw. Verdünnungsmittel kommen beispielsweise in Frage: Wasser, Ethanol, Methanol, Propanol, Isopropanol, Butanol, Aceton, Dimethylketon, Methylethylketon, Dimethylacetamid, Dimethylformamid, N-Methylpyrollidon, Acetonitril, Pentan, Hexan, Heptan und Gemische davon. Bevorzugt sind Ethanol, Isopropanol oder Acteon. Zu bevorzugen ist ebenfalls ein Wassergehalt von 1 bis 60 %, vorzugsweise von 5 bis 50%.

Zu der Lösung bzw. Suspension des Ausgangspeptidsalzes wird der Mischbettionenaustauscher, also ein Gemisch von einem sauren und einem basischen Ionenaustauscher gegeben. Als Ionenaustauscher kommt beispielsweise Amberlite ® in Frage.

Die Menge an Ionenaustauscher richtet sich nach der Anzahl der basischen Gruppen pro Peptid. Die Menge wird durch Zugabe bis Erhalt eines konstanten ph-Wertes ermittelt. Beispielsweise werden für 1g Cetrorelix 10g Amberlite MB-3 benötigt.

Die pH-Werte der Basenlösungen bei der Basenherstellung stellt sich je nach eingesetztem Wirkstoff-Salzes, insbesondere bei Peptidsalze mit basisch reagierenden Aminosäuren insbesonders jedoch bei Salzen von LHRH-Antagonisten (beispielsweise Cetrorelix, D-63153, Abarelix, Ganirelix, Ramorelix welche beispielsweise als Acetat vorliegen können), je nach verwendetem Wirkstoff, auf 7,5 bis 13 ein.

Die Temperatur sollte 25-30 °C nicht übersteigen, um einen Abbau des Peptids zu vermeiden. Die Reaktionszeit für die Herstellung der freien Base erfolgt üblicherweise innerhalb von wenigen Minuten, beispielsweise 20 min ausgehend von Cetrorelixacetat. Sie kann auch länger sein, beispielsweise etwa 1 h ausgehend von Cetrorelixembonat. Nach Erreichen eines konstanten pH-Wertes sollte die Reaktion abgebrochen werden da sich beispielsweise Zersetzungsprodukte aufgrund der Basizität der Lösungen bilden können.

Anschließend wird der Ionenaustauscher von der Reaktionsmischung entfernt. Die Entfernung kann durch Absieben, Abfiltrieren, Abzentrifuguieren oder Säulenfiltration geschehen.

Die klare bis trübe Lösung der freien Peptidbase, welche instabil ist, sollte möglichst rasch mit der Säure unter Bildung des gewünschten Säureadditionssalzes umgesetzt werden. Die Zugabe der Säure kann als Festsubstanz oder in Lösung bzw. als Suspension erfolgen. Genauso kann die Lösung der freien Peptidbase zur Säure erfolgen.

Die Reaktionszeiten liegen im Bereich von einigen Minuten bis einigen Stunden. Beispielsweise für die Bildung von Cetrorelix-Embonat beträgt die Reaktionszeit 1,5 h.

Anschließend wird die Reaktionslösung, die üblicherweise klar ist, sterilfiltriert. Anschließend kann das Lösungsmittel entfernt werden unter Erhalt des reinen Peptidsalzes. Alternativ können vor dem Entfernen des Lösungsmittels Hilfs-, Zusatz- oder Trägerstoffe der Lösung zugesetzt werden. Die Zugabe der Hilfsstoffe kann als Feststoff vor der Sterilfiltration oder nach der Sterilfiltration als als sterilfiltrierte Lösung erfolgen.

Geeignete Hilfsstoffe sind beispielsweise Mannitol, Sorbitol, Xylit oder lösliche Stärke.

Erfindungsgemäß können folgende Salze durch Zugabe der entsprechenden Säure hergestellt werden: Acetat, Adipat, Ascorbat, Alginat, Benzoat, Benzolsulfonat, Bromid, Carbonat, Citrat, Chlorid, Dibutylphosphat, Dihydrogencitrat, Dioctylphosphat, Dihexadecylphosphat, Fumarat, Gluconat, Glucuronat, Glutamat, Hydrogencarbonat, Hydrogentartrat, Hydrochlorid, Hydrogencitrat, Jodid, Lactat, alpha-Liponsäure, Malat, Maleat, Malonat, Pamoat (Embonat), Palmitat, Phosphat, Salicylat, Stearat, Succinat, Sulphat, Tartrat, Tannate, Oleat oder Octylphosphat

### Beispiel:

46,47 g D-20761 wurden portionsweise zu 1193 g Wasser gegeben und unter Rühren gelöst. (= Lösung 1). Lösung 1 wurde anschließend unter Rühren mit 3261 g 96 % Ethanol verdünnt. (= Lösung 2). Lösung 2 wurde nach dem Verdünnen über einen Glasfaservorfilter filtriert und das Filtrat mit 390 g Amberlite MB3 (Mischbettionenaustauscher aus stark sauren Kationen und Anionenaustauschern) unter Rühren versetzt (= Mischung 1).
316,8 g Mannit wurde in 1267 g Wasser unter Rühren gelöst.(=Lösung 3).

Nach 15 min. Rühren, wurde der pH-Wert der überstehenden Lösung von Mischung 1 gemessen, und nach weiteren 5 minütigem Rühren wurde nochmals der pH-Wert gemessen. Die Lösung wurde anschließend nach erreichtem pH-Wert von 12,5 über ein feinmaschiges Sieb vom Amberlite MB3 abgetrennt. (= Lösung 4).
4162 g der Lösung 4 wurde mit 5,34 g Embonsäure unter Rühren versetzt. Diese Mischung wurde weitere 1,5 h kräftig gerührt und die etwas trübe Lösung anschließend über ein Glasfaservorfilter filtriert. Bei dieser Lösung wurde ein pH-Wert von 8,4 (= Lösung 5). Die messenen pH-Werte wurden mit einer Schliffelektrode gemessen mit einer viskosen Elektrolytflüssigkeit. Die pH-Werte waren nur als Relativwerte zu sehen, da die gemessenen Lösungen bzw. Suspensionen Ethanol enthalten und somit einen scheinbar höheren Werte anzeigten.
3333 g der Lösung 5 wurden in die auf RT temperierte Reaktionsapparatur sterilfiltriert und 528 g der Lösung 3 zu der auf RT temperierten Lösung 5 unter Rühren sterilfiltriert.(= Lösung 6).
Lösung 6 wurde auf 40 °C erwärmt und anschließend das Ethanol / Wassergemisch bis auf <= 1931 g i.Vak. abdestilliert. (= Suspension 1). Die Cetrorelix-Embonat-Suspension 1 wurde auf RT abgekühlt und mit sterilfiltriertem Wasser für Injektionszwecke ad 3000 g unter Rühren verdünnt.(= Suspension 2). Die fertige, auf RT temperierte Suspension 2 wurde anschließend zu je 3,0 g in 10 ml Injektionsflaschen abgefüllt, mit Gefriertrocknungsstopfen versehen und in die Gefriertrocknungsanlage überführt.
Die Injektionsflaschen wurden bei einer Plattentemperatur von -40° C in einer Gefriertrocknungsanlage eingefroren. Die Trocknung erfolgte mittels eines Trocknungsprogramms bei einer Plattentemperatur von -40° C auf 20° C steigend. Die GT-Anlage mit wurde mit sterilfiltriertem Stickstoff geflutet, die Injektionsflaschen in der Anlage verschlossen und anschließend die Bördelkappen aufgesetzt und rolliert.
Nach der Gefriertrocknung wurden die verschlossenen Injektionsflaschen bei 12 kGy (min) - 15 kGy gamma-strahlensterilisiert. Letzteres ist optional. 1 Injektionsflasche zu 140,0(7) mg enthält 34,07 mg Cetrorelix-Embonat entsprechend 30 mg Cetrorelix.
und 106 mg Mannitol. Zur Rekonstitution werden 2 ml Wasser für Injektionszwecke verwendet. Die erhaltene Suspension kann per i.m. oder s.c. Gabe verabreicht werden.

### Biologische Wirkung:

Das gemäß Beispiel 1 erhaltene Cetrorelix Embonat (2:1) Lyophilisat (30 mg) wird in 2 ml Wasser für Injektionszwecke resuspendiert und kann dann parenteral, vorzugsweise subkutan (s.c.) oder intramuskulär (i.m.) verabreicht werden.

Bei s.c. Verabreichung findet man für das Cetrorelix Embonat (2:1) eine Bioverfügbarkeit von ca. 30-50 % (100% = intravenös verabreichtes Cetrolixacetat). Ein besonderer Vorteil von Cetrorelix Embonat (2: 1) Lyophilisat ist der geringe bzw. kein sog. Burst-Effekt beim Patienten. Die Wirkungsdauer ist dosisabhängig und liegt bei einer Dosis von 30-150mg bei 2-8 Wochen oder länger. Das erfindungsgemäße Cetrorelix-Embonat (2:1) Lyophilisat ist bereits in Klinikphase 1 am Menschen untersucht worden.

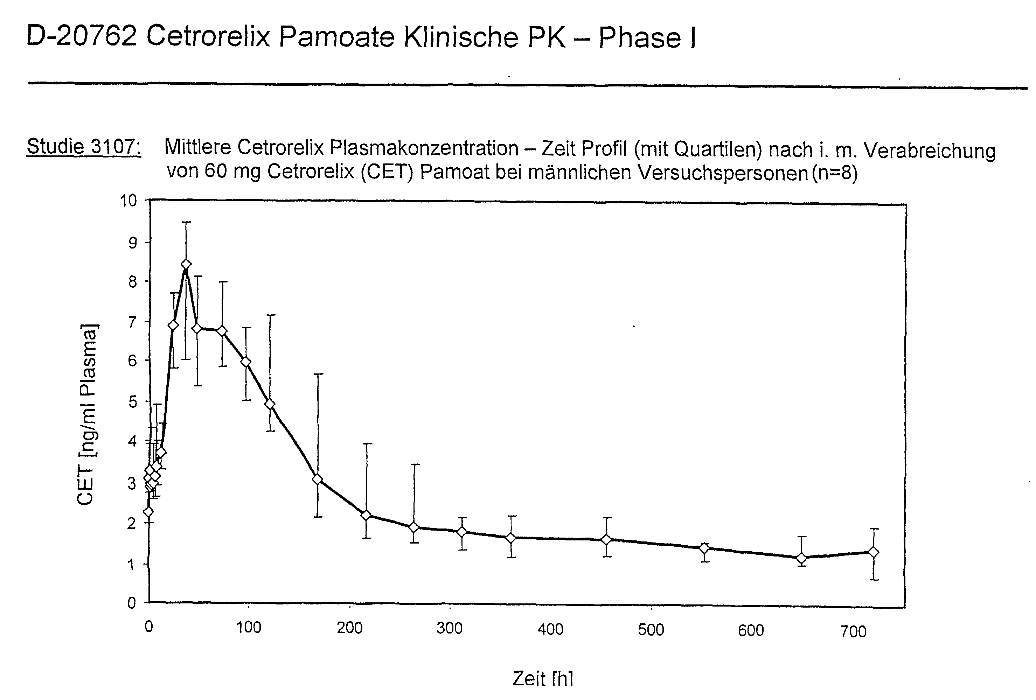

Fig. 1 zeigt Den Verlauf der Cetrorelix-Plasmakonzentration in Abhängigkeit von der Zeit (in Stunden) beginnend ab der i.m. Gabe von 60 mg Cetrorelix-Embonat (2:1)-Lyophilisat gemäß Beispiel 1 beim Menschen. Kein Burst-Effekt (ca. 100ng/ml) feststellbar. Wirkungsdauer mehr als 700 Stunden. Ab 150 h nach Verabreichung konstanter Plasmaspiegel von ca. 2 ng/ml. Die Bioverfügbarkeit lag bei ca. 40 %.

Die Anwendungsgebiete der erfindungsgemäßen Peptidsalze sind beispielsweise BPH, Myoma und Endometriose.

## Patentansprüche

1. Verfahren zur Herstellung einer pharmazeutischen Zubereitung zur parenteralen Applikation bei Säugetieren, **dadurch gekennzeichnet, dass** ein leichtlösliches Säureadditionssalz eines basischen peptidischen LHRH-Antagonisten in Gegenwart eines geeigneten Verdünnungsmittels mit einem Mischbettionenaustauscher oder einem Gemisch von einem sauren und einem basischen Ionenaustauscher unter Bildung des freien basischen Peptids umgesetzt, anschließend der Ionenaustauscher abgetrennt und dann das freie basische Peptid mit einer anorganischen oder organischen Säure unter Bildung des gewünschten schwerlöslichen Säureadditionssalzes des Peptids umgesetzt wird, dann geeignete pharmazeutische Hilfs-, Träger und/oder Gerüststoffe zugesetzt werden und anschließend das Verdünnungsmittel entfernt wird.

2. Verfahren nach Anspruch 1, **dadurch gekennzeichnet, dass** ein Salz von Cetrorelix, Teverelix, Abarelix, Ganirelix, Azaline B, Antide, A-75998, Detirelix, Ramorelix oder RS-68439 als Ausgangspeptidsalz eingesetzt wird.

3. Verfahren nach Anspruch 1 oder 2, **dadurch gekennzeichnet, dass** die Säure Embonsäure, Stearinsäure oder Salicylsäure ist.

4. Verfahren nach einem der Ansprüche 1 bis 3, **dadurch gekennzeichnet, dass** das Molverhältnis Cetrorelix zu Embonsäure 2 : 1 beträgt.

5. Verfahren nach einem der Ansprüche 1 bis 4, **dadurch gekennzeichnet, dass** das Verdünnungsmittel durch Gefriertrocknung entfernt wird.

## Claims

1. Process for the production of a pharmaceutical preparation for parenteral administration to mammals, **characterized in that** an easily soluble acid addition salt of a basic peptidic LHRH antagonist is reacted in the presence of a suitable diluent with a mixed bed ion exchanger or a mixture of an acidic and a basic ion exchanger with formation of the free basic peptide, the ion exchanger is subsequently separated off and the free basic peptide is then reacted with an inorganic or organic acid with formation of the desired poorly soluble acid addition salt of the peptide, suitable pharmaceutical excipients, vehicles and/or bulking agents are then added and the diluent is subsequently removed.

2. Process according to claim 1, **characterized in that** a salt of cetrorelix, teverelix, abarelix, ganirelix, azaline B, antide, A-75998, detirelix, ramorelix or RS-68439 is employed as the starting peptide salt.

3. Process according to claim 1 or 2, **characterized in that** the acid is embonic acid, stearic acid or salicylic acid.

4. Process according to one of claims 1 to 3, **characterized in that** the molar ratio of cetrorelix to embonic acid is 2:1.

5. Process according to one of claims 1 to 4, **characterized in that** the diluent is removed by freeze drying.

## Revendications

1. Procédé de production d'une préparation pharmaceutique pour application parentérale chez les mammifères,
**caractérisé en ce qu'**
un sel d'addition à un acide, facilement soluble, d'un antagoniste peptidique basique de l'hormone de libération de la lutéinostimuline, est mis en réaction, en présence d'un diluant adéquat, avec un échangeur d'ions à lit mixte ou un mélange constitué d'un échangeur d'ions acide et d'un basique, avec formation du peptide basique libre, ensuite l'échangeur d'ions est séparé et le peptide basique libre est mis en réaction avec un acide inorganique ou organique, avec formation du sel désiré d'addition à un acide, difficilement soluble, du peptide, puis des adjuvants, supports pharmaceutiques et/ou substances constituant une charpente sont ajoutés et ensuite le diluant est éliminé.

2. Procédé selon la revendication 1,
**caractérisé en ce qu'**
un sel de Cetrorelix, Teverelix, Abarelix, Ganirelix, Azaline B, Antide, A-75998, Detirelix, Ramorelix ou RS-68439, est utilisé comme sel de peptide de départ.

3. Procédé selon la revendication 1 ou 2,
**caractérisé en ce que**
l'acide est l'acide embonique, l'acide stéarique ou l'acide salicylique.

4. Procédé selon l'une des revendications 1 à 3,
**caractérisé en ce que**
le rapport molaire du Cetrorelix à l'acide embonique est 2:1.

5. Procédé selon l'une des revendications 1 à 4,
**caractérisé en ce que**
le diluant est éliminé par lyophilisation.
